# EUROPEAN PATENT SPECIFICATION

(11) **EP 2 621 503 B1**
(45) Date of publication and mention of the grant of the patent: **01.11.2017**
(21) Application number: 11829685.4
(22) Date of filing: 29.09.2011
(51) Int. Cl.: A23L 19/12, A23L 29/238, A23L 33/21, A23L 7/117, A23L 9/10, A21D 2/18, A21D 2/36, A61K 31/736

(54) **USE OF A FOOD PRODUCT COMPRISING VISCOUS FIBRES WHICH HAS A BENEFICIAL EFFECT ON COGNITIVE PERFORMANCE.**
VERWENDUNG EINES NAHRUNGSMITTELS MIT VISKÖSEN FASERN UND MIT EINER POSITIVEN WIRKUNG AUF DIE KOGNITIVE LEISTUNG
UTILISATION D'UN PRODUIT ALIMENTAIRE CONTENANT DES FIBRES VISQUEUSES ET AYANT UN EFFET BÉNÉFIQUE SUR LA PERFORMANCE COGNITIVE

(30) Priority: 29.09.2010 SE 1051011
(43) Date of publication of application: 07.08.2013
(73) Proprietor: Orkla Foods Sverige AB, 241 81 Eslöv (SE); Pågen AB, 200 41 Malmö (SE); Björck, Inger, 241 95 Billinge (SE); Nilsson, Anne, 257 33 Rydebäck (SE); Radeborg, Karl, 245 63 Hjärup (SE)
(72) Inventor: BJÖRCK, Inger, S-241 95 Billinge (SE); NILSSON, Anne, S-257 33 Rydebäck (SE); RADEBORG, Karl, S-245 63 Hjärup (SE)
(74) Representative: Brann AB
(86) International application number: PCT/SE2011/051160
(87) International publication number: WO 2012/044242

(56) References cited:
- EP-A1- 2 179 661
- WO-A1-2007/041817
- WO-A1-2007/041817
- WO-A1-2009/045481
- WO-A1-2009/082203
- A NILSSON ET AL: "Effects of differences in postprandial glycaemia on cognitive functions in healthy middle-aged subjects", EUROPEAN JOURNAL OF CLINICAL NUTRITION, vol. 63, no. 1, 12 September 2007 (2007-09-12), pages 113-120, XP055120185, ISSN: 0954-3007, DOI: 10.1038/sj.ejcn.1602900
- DIKEMAN C L ET AL: "Viscosity as related to dietary fiber: a review", CRITICAL REVIEWS IN FOOD SCIENCE AND NUTRITION, TAYLOR & FRANCIS, USA, vol. 46, no. 8, 1 January 2006 (2006-01-01), pages 649-663, XP008170211, ISSN: 1040-8398 [retrieved on 2007-06-18]
- CIOK J ET AL: "Carbohydrates and mental performance-the role of glycemic index of food products", MEDLINE,, 1 March 2006 (2006-03-01), XP003031866,
- WILLIAM WU ET AL: "The brain in the age of old: The hippocampal formation is targeted differentially by diseases of late life", ANNALS OF NEUROLOGY, vol. 64, no. 6, 1 December 2008 (2008-12-01), pages 698-706, XP055083580, ISSN: 0364-5134, DOI: 10.1002/ana.21557
- NILSSON A ET AL.: 'Effects of differences in postprandial glycaemia on cognitive functions in healthy middle-aged subjects' EUROPEAN JOURNAL OF CLINICAL NUTRITION vol. 63, no. 1, 2009, pages 113 - 120, XP055120185
- DIKEMAN C ET AL.: 'Viscosity as related to dietary fiber: a review' CRITICAL REVIEWS IN FOOD SCIENCE AND NUTRITION vol. 46, no. 8, 2006, pages 649 - 663, XP008170211
- DATABASE MEDLINE CIOK J ET AL.: 'Carbohydrates and mental performance-the role of glycemic index of food products', XP003031866 Database accession no. NLM16780278 & POLSKI MERKURIUSZ LEKARSKI : ORGAN POLSKIEGO TOWARZYSTWA LEKARSKIEGO vol. 20, no. 117, March 2006, ISSN 1426-9686 pages 367 - 370
- WU W. ET AL.: 'The brain in the age of old: the hippocampal formation is targeted differentially by diseases of late life' ANNALS OF NEUROLOGY vol. 64, no. 6, December 2008, pages 698 - 706, XP055083580

## Description

### Field of the invention

The present invention relates to a food product comprising viscous fibres and with specific glycaemic features which has a beneficial effect on cognitive performance and/or on metabolism in subjects with cognitive dysfunction secondary to insulin resistance and insulin resistance syndrome.

### Background of invention

Glucose is the main fuel of the brain, and optimal cognitive functions require maintenance of an adequate blood glucose level. Several studies have demonstrated positive effects on cognitive functions when providing glucose in the form of a glucose drink, compared with no glucose. A period of intensive cognitive demand has been shown to result in a measurable decrease in peripheral blood glucose levels in man, caused by increased neural energy expenditure. Since glucose is the main fuel of the brain, it can be hypothesised that differences in rate and duration of post-prandial glucose delivery to the blood after carbohydrate-rich foods could affect cognitive functions during the post-prandial phase. Only a limited number of studies have previously been performed in this research area, and as yet few studies have provided information of the glycaemic profile extending beyond the time frame used for calculating the Glycaemic Index (GI); which is based on glycaemic areas during 120 mins, or 90 mins post meal. It is evident from the literature that type-2 diabetes is associated with an increased risk of cognitive dysfunction. An accumulating body of data also shows a relation between glucose tolerance and cognitive functions in healthy adults, especially in elderly persons, with an impaired function in subjects with impaired glucose tolerance. Previously we have shown (Nilsson A, Radeborg K, Björck I (2009) Eur J Clin Nutr. Vol 63, 113-120) that subjects with higher glucose tolerance performed superiorly in the cognitive tests compared with subjects with lower glucose tolerance, despite the fact that all had glucose tolerance within the normal range.

The insulin resistance syndrome (IRS) includes a cluster of dysfunctions that identifies subjects at risk of developing type-2 diabetes and cardiovascular disease (CVD). Central to this syndrome is obesity, insulin resistance (IR), impaired glucose tolerance (IGT) and hyperglycaemia; but also e.g. dyslipidaemia, elevated blood pressure, imbalance in lipoproteins and pro-thrombotic factors, and a state of sub-clinical inflammation. Chronic hyperinsulinemia, a consequence of peripheral insulin resistance, results in a reduction of insulin transport across the blood-brain barrier and insulin resistance also in the brain. Insulin resistance is accompanied by reduced insulin signalling in brain, altering a variety of insulin mediated events of importance for memory functions. The IRS, IGT, and type-2 diabetes are increasingly being associated with impaired cognitive functions (Taylor V.H, MacQueen G. M, 2007 Obes Rev, Vol 8, 409-418), and cognitive dysfunction might thus be considered an additional feature of IRS. Hyperinsulinemia as well as type-2 diabetes also appears to promote Alzheimer's disease (de la Monte, S. M. 2009 BMB Rep, Vol 42, 475-478; Kroner 2009 Altern Med Rev, vol 14, 373-379). The link between insulin resistance and neurodegeneration thus indicate a new target for prevention and therapy.

By consuming carbohydrate foods of high glycaemic index (GI), post-prandial hyperglycaemia may trigger oxidative stress and inflammation, of which both negatively impact cardiovascular health. In contrast, low GI diets have been reported to facilitate dietary management of type-2 diabetes and related disorders, and also to prevent development of such diseases. In fact, in a recent review it was concluded that the most successful dietary strategies, with the strongest evidence of being able to prevent type-2 diabetes, are those that reduce post-prandial glycaemia and insulinaemia, without adverse effects on any other risk factors. A low GI diets is one example of such a strategy.

There is currently a shortage of low GI products on the market. Additionally, low GI food may vary in features of the very late post-prandial course of glycaemia.

It is known that carbohydrate foods vary in impact on peak blood glucose response and incremental blood glucose area up to 1.5 or 2h in the post-prandial phase, as judged from calculations of Glycaemic Indices (GI) using this time perspective. However, the over-all course of glycaemia is of importance, and a low GI may not necessarily coincide with a sustained net increment in blood glucose levels in the later part of the post-prandial phase (1.5 to 4 hours).

The document Nilsson A. et al: ("Effects of differences in postprandial glycaemia on cognitive functions in health middle-aged subjects") discusses the relationship between cognitive performance and food via the brain's main fuel, glucose. This document discloses mimicking low GI or high GI meals by giving glucose drink in bolus for 0-12 min or divided into 6 aliquots sipped under 150 min.

The dcument Dikeman et al. "Viscosity as related to dietary fiber: a review" discloses that the mean area under graph (AUG) for blood glucose in response to consumption of a liquid test meal containing glucose decreased upon addition of oat gum.

The document Janusz et al: "Carbohydrates and mental performance - the role of glycemic index in food products" discloses that breakfast intake improves the ability of concentration, reaction time, learning ability, mood and memory. The document Wu et al: "The brain in the age of old: the hippocampal formation is targeted by diseases of late life" discloses that that interventions that cause a decrease in blood glucose would be cognitively beneficial.

WO 2007/041817 A1) discloses that dietetic fibers affect the carbohydrate metabolism, especially in relation with the effect of its viscosity in reducing postprandial hyperglycaemia.

WO 2009/045481) discloses a composition for enhancing cognitive function. The composition includes long chain polyunsaturated fatty acids, nitric oxide releasing compounds, and medium chain triglycerides.

EP 2 179 661 A1 discloses a biscuit comprising guar gum in rod-like form for use in diet of subjects suffering from diabetes and especially type 2 diabetes or insulin resistance.

WO 2009/082203 discloses a liquid nucleotides/nucleosides-containing product. Dietary fibers are included in the product to solve the problem how to avoid the formation of sediment in the product.

### Summary of the Invention

The above-mentioned deficiencies in the prior art have been overcome by means of the products, as defined in the appended set of claims.

In a first aspect the present invention is directed to a food product for use in preventing cognitive dysfunction secondary to Insulin Resistance and Insulin Resistance Syndrome in a subject, which upon ingestion leads to an elevation in delta blood glucose level of > 0 mmol/L in the interval 120 mins to 240 mins in the post-prandial phase, the food product having a G1 below 70 using a white wheat bread reference, and comprising viscous dietary fibre capable of reducing the rate of digestion and adsorption of carbohydrates in the gastrointestinal tract.

In a second aspect the invention relates to a food product for use according claim 1, wherein the food product comprises 5-35%, such as 10-20% viscous dietary fibre by dry weight.

In a third aspect the invention is directed to a food product for use according to claim 1 or 2, wherein the viscous dietary fibre is selected from the group consisting of pectins, beta-glucans, gums such as xanthan gum, guar gum and carob gum, arabinoxylans, alginates, glucomannans including konjacmannan, psyllium and carrageenans, and/or combinations thereof.

In a fourth aspect the invention is directed to a food product for use according to claim 3, wherein the viscous dietary fibre is guar gum.

The invention also relates to a food product for use according to claim 1, wherein the food product comprises 15% guar gum by dry weight.

In another aspect the food product for use according to any of the preceeding claims is selected from the group consisting of soft bread, crisp bread and flatbread, such as white bread, rye bread, whole wheat bread, whole grain bread or combinations thereof, cakes, buns, biscuits, pizza, pies, sauces, dressings, breakfast cereal products such as flakes and muesli, porridges, pasta, yoghurts, gel foods, snacks, bars, smoothies, shots, probiotic products, soups, drinks, drink or soup mix powders, shakes, fruit purees, composite ready-to- eat meals, lasagne, crepes, pancakes, flatbread wraps, pirogues, purees, jams, marmalades, puddings, jellies, mashed potatoes, and gratin or casseroles, for example potato gratin, broccoli gratin.

In a specific embodiment the food product for use according to any of the preceding claims is a bread.

In another embodiment the food product for use according to claim 1 is a cereal-based bread comprising guar gum.

### Brief Description of the Drawings

Figure 1 illustrates the design of Study 1 "Cognitive functioning in relation to post-prandial blood glucose profile".
Figure 2 depicts the Post-prandial blood glucose increments after four different test breads consumed in the morning after over-night fasting, n=12.
Figure 3 shows the results of the cognitive tests. A: the performance in the SA-test at 120 mins after breakfast was significantly better following the white bread with guar gum (WWB-gg) compared with a white bread without guar gum (WWB). B: When the results of the second half of the SA tests were analysed, the results showed a significant improved performance after the guar bread during the entire post-prandial period. C: Reaction time. D: results in the WM-tests.
Figure 4 shows the results in the cognitive tests in relation to the individual glucose tolerance, determined from the size of the incremental area under the 90 mins glucose curve after an oral challenge with 50 g glucose.
Figure 5 Flow distance in a Bostwick consistometer (n=2). The figure shows the flow velocity of three samples resembling different physiological conditions of the gastro-intestinal system. ●= mouth; ▲= stomach; ◆= small intestine.
Figure 6 Viscosity curve (n=2). The figure shows the viscosity curves for the three different samples. ●= mouth; ▲= stomach; ◆= small intestine.
Figure 7 Flow curve (n=2). The figure shows the flow curve for the three different samples. ●= mouth; ▲= stomach; ◆= small intestine.
Figure 8 shoes the flow curve for a comparison of WWB-gg and Guar gum suspended in water. ■= Guar Gum; ◆= WWB-gg.

### Definitions

For the purpose of this description, the following terms have the meanings ascribed to them.

The term "post-prandial" is used interchangeably with "post meal" and "post-prandial phase", where all these terms refer to a period of time starting immediately after ingestion of food, and onwards. Often the post-prandial phase is specified, e.g. 240 mins, which means the period of time immediately after ingestion of the meal and up to 240 mins after ingestion.

The term "delta blood glucose" refers to the difference in blood glucose levels in a subject between the level directly prior to ingestion of a meal, and the level at a post-prandial point in time.

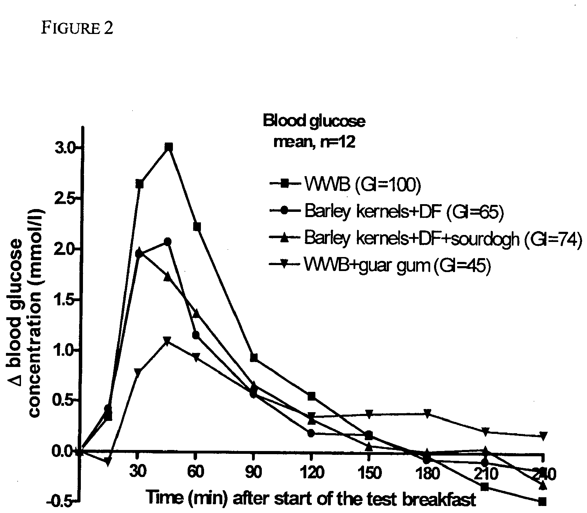

The term "post-prandial blood glucose profile" is intended to mean the curve received when delta blood glucose measurements are plotted against the time period in the post-prandial phase, for example for 240 mins in the post-prandial phase. An example of post-prandial blood glucose profiles for different food products is shown in figure 2.

The term "Viscous dietary fibres" refers to fibres that contributes to viscosity in the gastro-intestinal tract; that is maintain viscous properties following degradation of protein, starch and other digestible food components. The viscous dietary fibre polymers are commonly recovered in the "soluble" dietary fibre fraction. Viscous dietary fibre reduces rate of gastric emptying and gastric motility, and increases unstirred layer thus contributing to a reduced rate of digestion and absorption of available carbohydrates.

The term "Low molecular weight carbohydrates " are defined as those remaining soluble after enzymatic digestion of protein and starch followed by precipitation with 4 volumes 95% ethanol, reaching a final ethanol concentration of 78% (v/v) as described by Asp et al 1983 J Agric Food Chem.

The term "probiotics" refers to microorganisms which when administered in adequate amounts are established in the gastro-intestinal tract and considered to confer a health benefit to the host. Examples of probiotics are strains of lactic acid bacteria (LAB) e.g. *Lactobacillus acidophilus, Lactobacillus rhamnosus, Lactobacillus Plantarum; bifidobacteria* e.g. *Bifidobacteria lactis*, and combinations thereof. The probiotic may be supplied in solution or freeze-dried.

The term "Insulin Resistance Syndrome (IRS) refers to a cluster of dysfuntions and metabolic risk factors which identifies individuals with increased risk of type-2 diabetes and cardio-vascular disease.

The term "Insulin resistance (IR) refers to a condition with impairment of insulin receptor signalling and a condition of impaired ability for glucose regulation.

The term "Insulin sensitivity" refers to a measure of degree of insulin action, with an insulin sensitive condition corresponding to a normal insulin receptor signalling and normal glucose metabolism.

The term "Impaired glucose tolerance (IGT)" refers to a pre-diabetic condition which is characterized by lowered insulin sensitivity fasting state, and/or post-prandial blood glucose responses above normal following a glucose challenge.

The term "Hyperinsulinemia refers to a condition with elevated insulin secretion.

The term GI refers to Glycaemic Index, that is the post-prandial glycaemic response (incremental glycaemic area) to a carbohydrate test product expressed as a percentage of the corresponding response (incremental glycaemic area) with a carbohydrate equivalent amount of a reference product or pure glucose. In the literature GI refers to a timeperiod up to 1,5 or 2 hours post meal. With a white wheat bread as reference product, GI values are approximately 38% higher than with pure glucose as reference. The GI values presented in the present application have been obtained using a white wheat bread as a reference product.

The term "Low GI" refers to low Glycaemic Index, which is a low post-prandial glycaemic response to a carbohydrate test product compared to the corresponding response with a carbohydrate equivalent amount of a reference product or pure glucose. Low GI in the context of this application are GI values below 70.

The term "High GI" refers to high Glycaemic Index, that is a high post-prandial glycaemic response to a carbohydrate test product compared to the corresponding response with a carbohydrate equivalent amount of a reference product or pure glucose. High GI in the context of this application are values that are 70 and above.

The term "cognitive performance" is intended to mean the mental- and neuro-physiological processes of the brain. The quality of the cognitive performance may be measured by tests, such as those described herein (see e.g. Working Memory and Selective Attention test under Example 2). Improved cognitive performance may be determined by such tests and corresponds to the ability to answer correctly, and/or quickly in such tests.

### Detailed description

It has surprisingly been found that the products of the invention lead to increased delta blood glucose levels in the period of 120 - 240 mins post-prandially and to improved cognition in subjects.

The present invention relates to products which elicit such increased delta blood glucose levels, and use of said products to improve cognitive performance in subjects with cognitive dysfunction secondary to insulin resistance and insulin resistance syndrome.

Without being bound by theory, it is hypothesized that said foods exert positive effects on cognitive performance in two ways; by preventing cognitive dysfunction secondary to IR and IRS, and by facilitating cognitive performance due to a more favourable post-prandial course of glycaemia. The food product leads upon ingestion to an elevation in delta blood glucose level of > 0 mmol/L in the interval 120 mins to 240 mins in the post-prandial phase.

The blood glucose profile of the foods of the invention may be characterised by the fact that the delta blood glucose concentration at 2 hrs post-prandially is essentially the same as at 3 hrs post-prandially, and is maintained above 0 mmol/L between 2 to 3h, 2 to 4 or 2 to 5 hours post-prandially, or is maintained above 0 mmol/L in the interval between 3 to 4 h, or 4 to 5 h.

The food product of the invention has a GI below 70 (white wheat bread reference), such as GI= 25, GI= 30, GI= 40, GI=45, GI= 50, GI= 55, GI= 60, GI=65, or GI from 25 to 65, or GI from 25 to 55, or GI from 25 to 30, or GI from 30 to 45, GI of about 45, GI from 40 to 50, GI from 30 to 45, GI from 25 to 55, GI between 45 and 55, or GI between 50 and 60, or GI between 55 and 65, or GI between 60 and 70; and in addition produces a delta blood glucose level > 0 mmol/L for at least 3 hrs after meal, such as for 3,5 hrs after meal, for 4 hrs after meal, for 4,5 hrs after meal, or for 5 hrs after meal. The food product comprises viscous dietary fibre capable of reducing the rate of digestion and absorption of carbohydrates in the gastro-intestinal tract, thus producing a low and sustained net increment of blood glucose after a meal.

The viscous dietary fibre may be selected from the group consisting of viscous dietary fibre derived or extracted from fruit, vegetables, legumes or cereals; exo-cellular polysaccharides produced by microorganisms; alginates; and combinations thereof. Examples of viscous dietary fibre are pectins; beta-glucans, gums such as xanthan gum, guar gum and carob gum, arabinoxylans, alginates, glucomannans including konjacmannan, psyllium and carrageenans, and/or combinations thereof. In one embodiment the food product of the invention comprises guar gum.

The food product of the invention may comprise at least 5% viscous dietary fibre by dry weight, such as 5, 6, 7, 8, 9, 10, 11, 12, 13, 14, 15, 16, 17, 18, 19, 20, 21, 22, 23, 24, 25, 26, 27, 28, 29 or 30% by dry weight. Other examples include from 5 to 6%, to 12%, from 8 to 16%, from 12 to 24 %, from 13 to 18%, 15%, or 5 to 35% or for example 5- 15%, 10-30%, 15-30%, 15-25%, such as 10% to 20%, 10-20%, 20-35% or 20-30% viscous dietary fibre by dry weight. The food product comprises from 5 to about 35% or for example from 10 to 20% viscous dietary fibre by dry weight. Other examples comprise 10, 11, 12, 13, 14, 15, 16, 17, 18, 19, 20, 21, 21, 22, 23, 24, 25, 26, 27, 28, 29 or 30% viscous dietary fibre by dry weight. Another specific example is a food product, which comprises 18% total dietary fibre by dry weight, where 13% by dry weight consists of soluble and viscous dietary fibre. Thus, 13 of the 18 percent fibres by dry weight are made up of viscous fibres. In one example, the food product comprises 15% dry weight of guar gum.

The dietary fibre of the food product may be added or intrinsic to the ingredient material e.g. from cereal, legume, fruit, tubers or vegetables. The food structure may further be maintained such to preserve the botanical structure, hence lowering glycaemia.

The food product of the invention comprises at least one viscous dietary fibre, wherein the fluidity of said fibre is determined by the method described in Example 5. Said fibers may have a fluidity 0,3 to 0,5 Arbitrary Bostwick units, such as 0,3 to 0,5, such as 0,36 to 0,49, such as 0,35 to 0,47, such as 0,32 to 0,43, such as 0,37 to 0,43; or for example 0,3 to 0,4; for example 0,4, such as 0,41, such as 0,4 such as 0,39; or for example 0,41 to 0,43, such as 0,41 to 0,42 Arbitrary Bostwick Units. Said at least one viscous dietary fibre may have a fluidity of for example 0,32, 0,33, 0,34, 0,35, 0,36, 0,37, 0,38, 0,39, 0,40, 0,4, 0,41, 0,42 or 0,43 Arbitrary Bostwick Units.

The food product of the invention comprises at least one viscous dietary fibre, wherein said fibers may have a fluidity as measured by Bostwick consistometer and with a procedure as described in Examples 3 and/or 5 of for example from 0,4 to 0,36 Arbitrary Bostwick Units (ABU) at 60s at concentrations between 0,45 ml to 0,65 ml; or for example 0,37-0,33 ABU at 0,2 -0,35 g/100 ml, or for example 0,49 to 0,42 ABU at 1,5 to 2,5 g/100 ml; or for example 0,43-0,37 ABU at 4,0 to 5,0 g/100 ml, or for example 0,47 to 0,39 at 0,75-0,95 g/100 ml. The food product may comprise modified food structures capable of reducing the rate of digestion and absorption of carbohydrates in the gastro-intestinal tract, thus producing a favourable course of glycaemia with a low and sustained net increment of blood glucose after a meal.

Such modifications may obstruct the access of available carbohydrates for digestion and absorption, and in the case of starchy foods reduce access of starch to amylases (obstructed starch hydrolysis). Examples of such modifications include low gelatinization, high degree retrograde starch (may be accomplished for example by baking using the pumpernickel method), preservation of the botanical structure, thus encapsulating the dietary carbohydrates, mechanically encapsulated carbohydrates as for example in pasta, formation of lipid-starch interactions for example by adding monoglycerides, lowering of amylolysis through addition of amylase inhibitors, or preservation of intrinsic amylase inhibitors, introducing carbohydrate-protein interactions (for example with gluten, also e.g. found in pasta, or formed by adding lactic acid), addition of acids which slow the emptying of the stomach Examples of preserved botanical structures are inclusion of whole grains and/or seeds, such as flaxseed. A further example of such a modification is inclusion of soya fibre, thus in one example the food product comprises soya fibre. The effect of these structures on the blood glucose profile may be checked using an in vitro method for predicting GI by measurement of the rate of carbohydrate hydrolysis and/or release (Leeman, Bårström, Björck 2005 J Sci Food Agric Vol 85, 751-756). The food product may comprise both one or more viscous fibres and one or more modified food structures. The food product may in addition to the one or more viscous fibres and/or the one or more modified food structures also comprise additional components.

The food product of the invention may comprise one or more additional components selected from the group consisting of indigestible carbohydrates, polyphenols, fatty acids and probiotics.

One group of additional components consists of components which promote colonic fermentation. Thus, the food product may comprise as an additional component indigestible carbohydrates which are readily fermented in the colon such as e.g. low molecular weight carbohydrates, capable of improving insulin resistance in post-prandial phase.

It has been shown that colonic fermentation of indigestible carbohydrates such as dietary fibre and resistant starch may improve insulin sensitivity in the perspective from a late evening meal consisting of boiled or baked barley kernels, to a subsequent breakfast ingested 10h after the evening meal (Nilsson et al 2008 J Nutr., Vol 138, 732-739). A similar observation of benefits on glucose tolerance/insulin sensitivity has been observed in the perspective from a barley kernel based breakfast meal rich in indigestible carbohydrates, to an evening meal ingested 10h after breakfast (Nilsson et al 2008 Am J Clin Nutr., Vol 87, 645-654). Additionally, low GI features at one meal may improve glucose tolerance at the meal ingested immediately thereafter, or after 4h. Consequently, a low GI breakfast consisting of pasta improved glucose tolerance and lowered tri-glycerides at a subsequent standardized lunch meal, compared with a matched high GI white wheat bread breakfast (Liljeberg, Björck 2000 Eur J Clin Nutr., Vol 54, 24-28). It is thus known that low GI features and content of indigestible and fermentable carbohydrates may improve insulin sensitivity at proceeding meals.

The presently described intervention describes a concept whereby low molecular weight indigestible carbohydrates, by virtue of their more rapid fermentation in the colon may improve glucose tolerance/insulin sensitivity already in the late post-prandial phase following the acute meal. Products containing slowly digested and absorbed carbohydrates, and in addition containing indigestible but fermentable colonic substrates induce a low and prolonged glycaemic profile hence improving insulin sensitivity and cognitive functions in the late acute post-prandial phase.

Accordingly, the food products may comprise indigestible carbohydrates of low molecular weight which occur naturally in plants, such as for instance one or more of the following: inulin, lactulose, oligosaccharides including fructo-oligosaccharides (also known as oligofructose) and galacto-oligosaccharides, galacto-mannans, resistant starch or resistant starch dextrins, or combinations thereof. The oligosaccharides may also be produced from enzyme treatment and/or fermentation yielding indigestible carbohydrates of low molecular weight. The food products may comprise synthesised indigestible carbohydrates of lower molecular weight. Examples of these include: dextrinized dietary fiber polymers or resistant starch fractions. Such carbohydrates may be prepared by processes such as enzymatic hydrolysis or thermal treatment. Such dextrinised dietary fiber polymers and resistant starch fractions are available commercially for example, AXOS™, Hi-maize™ and Novelose™.

A second group of additional components are pro- and prebiotics. The food product may comprise the additional component of probiotics which may boost the prebiotic effect and rate of colonic fermentation. Examples of probiotics are strains of lactic acid bacteria (LAB), bifidobacteria, Escherichia coli, Saccharomyces cerivisae, and combinations thereof. Certain probiotics contain live cultures (e.g. in yoghurt) while others are freeze-dried.

A third group of additional components consists of polyphenols such as e.g. antho-cyanins, chlorogenic acid, caffeic acid, catechins, flavonoids, curcumin, reservatrol, dihydrochalcones and xanthone.

A fourth group of additional components consists of fatty acids rich in omega-3. The food product may relate to a suspension comprising viscous dietary fibres. Said suspension may be suitable for admixture with further ingredients and may be further processed to yield a food product of the categories mentioned above. The food product may be a cereal-based product. The cereal may be any cereal or combination of cereals suitable for the intended product category. Examples of cereals are maize, rice, wheat, barley, rye, oats, sorghum, millet. Wheat comprises all types of wheat, including but not limited to common wheat, triticale, durum, spelt and einkorn. The cereals present may be in any form, such as whole kernel, crushed, or flour; or in wet (suspension) or dry form. The food product may be a cereal-based product comprising viscous dietary fibre; wherein the cereal may be any one or combination of cereals, and the dietary fibre may be any single or combination of viscous dietary fibres. The food product or suspension may comprise wheat. In a preferred embodiment, the dietary fibre comprises guar gum.

In another preferred embodiment, the food product is a cereal-based bread, such as a cereal-based bread comprising guar gum. The food product may be a bread comprising viscous dietary fibre, and which results in GI under 50, such as a GI= 45, and sustained elevated blood glucose increments in the late post-prandial period. In a further embodiment, the food product of the invention is a bread comprising 13% soluble and viscous dietary fibre, based on dry weight.

Product categories that may be produced based on the invention are selected from the group consisting of soft bread, crisp bread and flatbread, such as white bread, rye bread, whole wheat bread, whole grain bread or combinations thereof, cakes, buns, biscuits, pizza, pies, sauces, dressings, breakfast cereal products such as flakes and muesli, porridges, pasta, yoghurts, gel foods, snacks, bars, smoothies, shots, probiotic products, soups, drinks, drink or soup mix powders, shakes, fruit purees, composite ready-to-eat meals, lasagne, crepes, pancakes, flatbread wraps, pirogues, purées, jams, marmalades, puddings, jellies, mashed potatoes, and gratin or casseroles, for example potato gratin, broccoli gratin. In one example the food product of the invention is a bread.

### Examples

### EXAMPLE 1. PRODUCT DEVELOPMENT AND EVALUATION OF GLYCAEMIA

A pilot study was performed prior to Study 1 with the purpose to develop two bread prototypes suitable for the study, i.e. breads which resulted in significantly different post-prandial blood glucose responses.
Four test products (breads) were developed:
- A white wheat flour bread (WWB)

### Recipe of white wheat bread (one loaf of bread)

The WWB was baked in a home baking machine (Severin model no: BM 3983, Menu choice; program 2 (white bread, 1000 g, quick (time: 2:35)). The bread was made from 540 g white wheat flour (Kungsörnen AB, Järna, Sweden), 360 g water, 4.8 g dry yeast, and 4.8 g salt.
- A white wheat flour bread supplemented with guar gum (WWB-gg). The bread consisted of 18% total dietary fibre (based on total dry weight) with 15 % (dry weight basis) coming from guar gum. The WWB-gg consisted of 13% soluble and viscous dietary fibre. (dry weight basis)

### Recipe of WWB-gg (one loaf of bread)

480g water (40°C)
400 g white wheat flour
71g guar gum (5000 cps/200 mesh, C.E. Roeper GmBH
Hamburg, Germany)
6.3 g Dry yeast
5 g NaCl

Mix the dry ingredients and add to the water during continued mixing. Knead the dough for 5 mins. Prove the dough for 30 mins in ambient temperature, knead for 1 min, form the bread and then let the dough prove for an additional 30 mins (37 °C). The bread was baked for one hour in a home baking machine (Severin model no: BM 3983), program no 12.
- A bread with whole meal rye flour and intact barley kernels (50% kernels, dry weight) supplemented with barley dietary fibres (20% total fibres, dry basis)
- A bread with whole meal rye flour and intact barley kernels (50% kernels, dry basis) supplemented with barley dietary fibres (19% total fibres, dry basis) and in addition also baked with sour dough.

### Fibre content of the breads

WWB-gg: Total dietary fibre (DF) 18 % (soluble 13 %, insoluble 5 %). The bread contains 15 % (dry weight) added guar gum.
WWB: Total dietary fibre (DF) 2,3% (soluble 1.9%, insoluble 0.4%).
The barley dietary fibres included in the bread were obtained from Lyckeby Stärkelsen AB Degerbergavägen 60-20; SE-291 91 Kristianstad.

The breakfast post-prandial (0-4 h) blood glucose profiles were determined after the four test products in fifteen healthy young adult subjects (BMI< 25). The test subjects consumed the products at different days separated by approximately 1 week.

The mean post-prandial blood glucose responses after the four test breads developed in the pilot study are shown in Figure 2. We found that we successfully have managed to develop two suitable bread products to be included in a cognitive study; one test product (white wheat bread with guar gum), and a reference product (white wheat bread). The white bread with guar gum (WWB-gg) resulted in a post-prandial blood glucose profile very adequate, i.e. low GI and sustained elevated blood glucose increments in the late post-prandial period. A post-prandial glucose profile as seen after the guar gum bread is in addition probably also beneficial with the respect to metabolic aspects, e.g. in prevention and treatment of diabetes type-2. Consequently, this bread was chosen as a test product for further studies, using a white wheat flour bread (WWB) as a high-GI reference product.

### Example 2-Cognitive study

### Study design (Study 1)

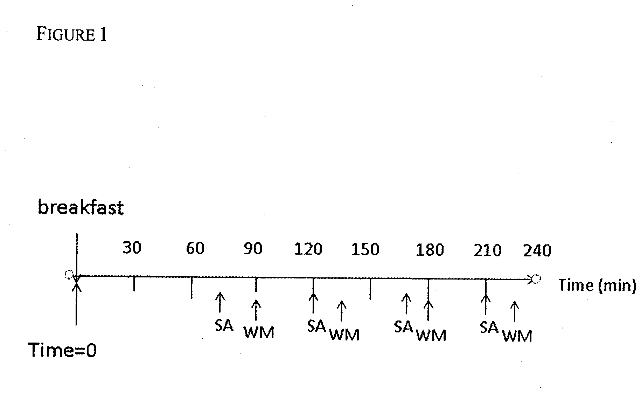

Healthy subjects (28 women and 12 men) with normal BMI (≤25) were included in the study. The study had a cross-over design i.e. the test subjects consumed both test products (separated by approximately one week), and consequently, the subjects constitute their one control. The subjects arrived fasting and consumed WWB reference or WWB-gg test breakfast prior to the performance of the cognitive tests. The cognitive tests consisted of four tests of working memory (WM; see Radeborg et al. (1999)) and four tests of selective attention (SA; previously developed in our lab (Nilsson, Radeborg, Björck, 2009;)), performed in the post-prandial period. Each test took approximately 10 mins to perform. The time periods in-between the performance of the cognitive tests were standardised in the way that the subjects performed Sudoku during all time periods in-between the tests. The study design during the test days is shown in figure 1.
Prior to the start of the study, the cognitive tests (WM tests and SA tests, respectively) had been optimized with the intention to be more sensitive to detection of differences depending on the test variables.

### Test of Working memory (WM)

The WM capacity was estimated by tests of the type originally used by Daneman and Carpenter (1980), requiring simultaneous storing and processing of information. The tests employed in the present study represent an extension of the methodology originally developed by Radeborg et al. (1999). There are several reasons for choosing WM as a measure of cognitive performance in this study. WM can be defined as a system responsible for simultaneous temporary storing and processing of information, and is involved in many everyday activities; such as mathematical problem solving where one often has to remember part of the result in a calculation while performing further mathematical operations. WM represent a fundamental ability for higher-level cognitive processes. Thus, measures of WM capacity have been shown to correlate significantly with activities as diverse as e.g. reading comprehension, note taking, following of directions, reasoning, and complex learning. Some authors even claim that WM and general problem solving ability or intelligence, as measured by e.g. Raven's Matrices, reflect nearly identical constructs. However, whereas intelligence tests generally only can be administered once due to risk of considerable learning effects, WM can be measured repeatedly, thus enabling one to study the development of WM over a period of time; or in this particular study in the post-prandial phase. The WM-tests in this study were oral, and each test composed of 12 sets of short declarative sentences (3-5 sentences, four of each number) that could be either semantically meaningful of the type 'the boy brushed his teeth', or nonsensical, such as 'the rabbit struck the idea'. The sentences were read one by one to the subjects, and immediately after each sentence they had to indicate if the sentence was semantically meaningful or not. The subjects were blind to the number of sentences in each set (3-5 sentences). After each set, the subjects had to repeat, in any order, the first noun in each of the four sentences. Four WM-tests were included at each experimental day. Eight different but comparable WM-tests were included in the study. The first test at each experimental day began with a short training session. One WM-test took approximately 10 mins to perform

### Test of Selective Attention (SA)

The test was based on spatial perception and primarily measured the ability to sustain a prolonged attention, and to control and split the attention to the entire picture on the computer screen. However, like the WM-test, this test also deals with simultaneous temporary storing and processing of information (working memory capacity). The storing time required was however shorter compared with the WM-test, whereas the time pressure was higher. The test is a further development of the test originally used in the study by Nilsson et al. (2009). The SA was measured using a computerized test made up of 96 pictures, each shown for two seconds on the screen. The pictures consisted of a square on a white back ground divided into four smaller squares. One of the smaller squares was red, one square was green, and two squares were uncoloured (white), resulting in a total of 12 unique picture combinations. The subjects had to remember the positions of the coloured squares, and to compare each new picture with the preceding one. Each time a new picture emerged on the screen, either the green, the red or none of the coloured squares were positioned in the same position compared with the previous picture. Within two seconds, the subjects were supposed to indicate by pressing one of three different keys on the keyboard, which of the three possible alternatives that occurred for each new picture. The test began with a short training session, and took approximately 10 mins to perform. The test was scored with the number of correct responses (CR, total 95 credits) and for the reaction time (RT) needed to give the answer (i.e. press one of the keys).

### Results

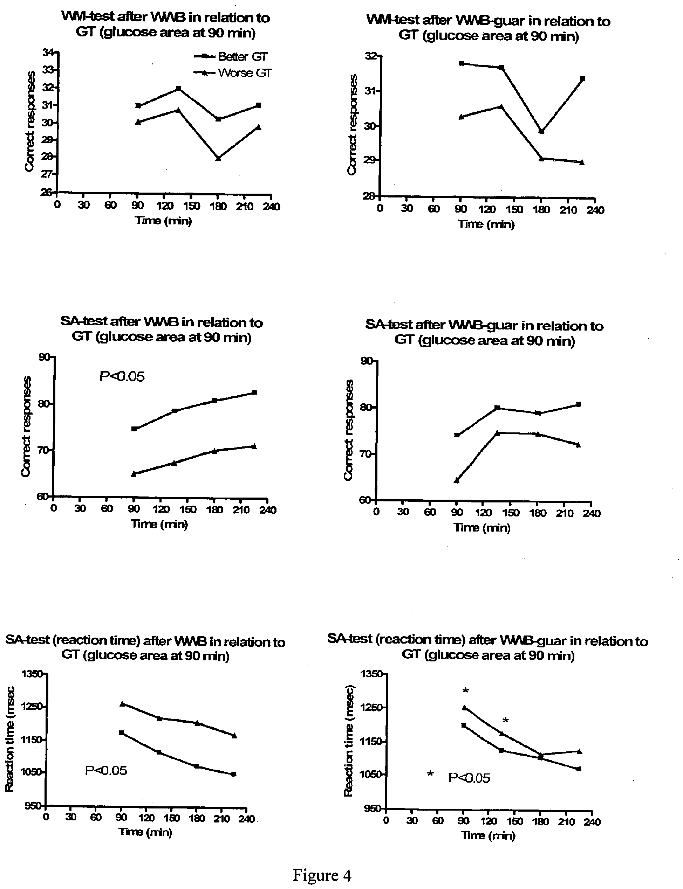

The results show that the cognitive performance (SA-test) at 2h in the post-prandial period was significantly improved after the guar gum bread (WWB-gg) breakfast, compared with after the white wheat reference bread without guar gum (WWB-ref) (P<0.01). Additionally, in the most demanding part of the SA test, that is in the later part of the computerised test where the need for focus is most challenging, a main effect of breakfast was observed in favour of the WWB-gg bread (P <0.01) over the entire test period from 75 to 220 mins. Moreover, the influence of differences in glucose tolerance on SA score was less pronounced in the case of the WWB-gg breakfast, as manifested by less differences in between the graphs for "better" vs "worse" Glucose Tolerance (GT) in the two lower panels of figure 4.

The invented product therefore infer additional benefits for subjects with lower glucose tolerance, in this case still within the normal range (see definitions of better and worse GT below).

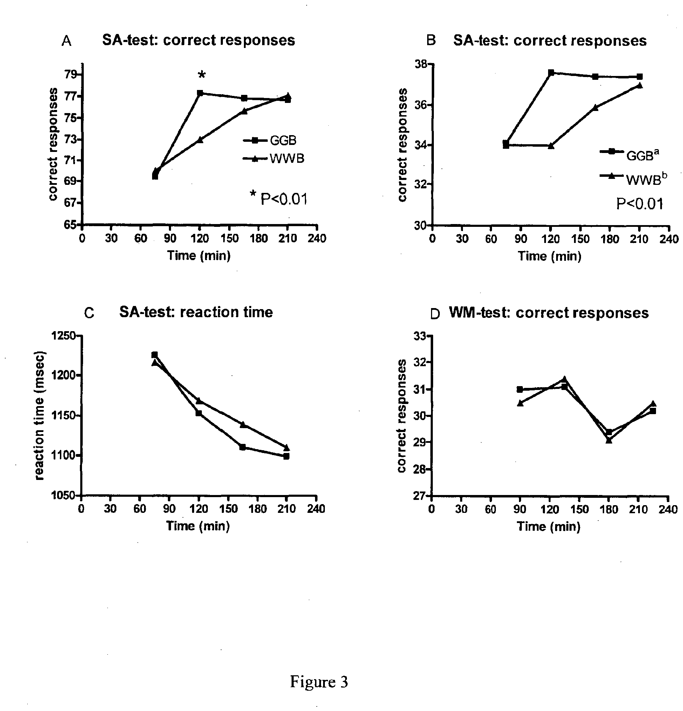

At 75 mins, when commencing the cognitive test battery, there was a steep fall in blood glucose concentrations after the WWB-ref compared with the WWB-gg, which probably deteriorates the cognitive performance. At two hours and 45 mins after the breakfast there was a tendency towards a faster reaction time after WWB-gg in comparison with the WWB-ref (P<0.074). There were no significant differences in the WM-test depending on the test breakfasts. The results of the cognitive tests are shown in figure 3.

The subjects were divided into two groups (20 + 20 subjects) according to how they responded in blood glucose concentrations to a 50 g glucose solution. The post-prandial glucose areas were determined and the median area was calculated. The 20 subjects with larger glucose area compared to the median area were classified as with "lower glucose tolerance", and the 20 subjects with a smaller glucose area were classified as with "higher glucose tolerance". As a general feature, the subjects with lower glucose tolerance (GT) performed less well in the cognitive tests compared with the subjects with higher GT, both in the SA-tests and in the WM-tests (Figure 4, P < 0.05).

### Example 3: Rheological characterisation of bread enriched with Guar Gum.

Two different methods were used in the characterization of the product; Fluidity using a Bostwick consistometer and viscosity using a Rheometer.

### Fluidity of the bread

5,37 g bread (corresponding to 1,5 g available starch) was weighed, wetted with 5-10 ml phosphate buffer and ground using 15 strokes during 15 seconds. A further 10-15 ml phosphate buffer was added (total volume of 20 ml) and the sample was ground for a further 20 s. Thereafter three samples according to the following were prepared.
1. Sample resembling physiological conditions in the small intestine: Ground sample was mixed with 29,75 ml phosphate buffer and 1,5 ml pepsin solution (30 U), the pH was adjusted to 1,5 with 2 M HCl.The sample was incubated for 30 mins in a water bath at 37°C, the pH adjusted to 6,9 with 2 M NaOH and 0,75 ml α-amylase (165 U) was added, the sample was incubated for an additional hour (37°C).
2. Sample resembling physiological conditions of the stomach:
   Ground sample was mixed with 30,5 ml phosphate buffer and 1,5 ml pepsin solution (30 U), the pH was adjusted to 1,5 with 2 M HCl. The sample was incubated for 30 mins in a water bath at 37°C.
3. Sample resembling the bread before it reaches the stomach (characteristics as in the mouth):
   Ground sample was mixed with 32 ml phosphate buffer.

The total volume of each sample used for fluidity measurements resembling physiological conditions in mouth, stomach, and small intestine, respectively, was 52 ml. 45 ml samples were used in the Bostwick consistometer, as described below. The amount of bread for incubation provided 1,5 g bioavailable starch and was chosen such that the sample should have optimal flow characteristics for the chosen measuring systems.

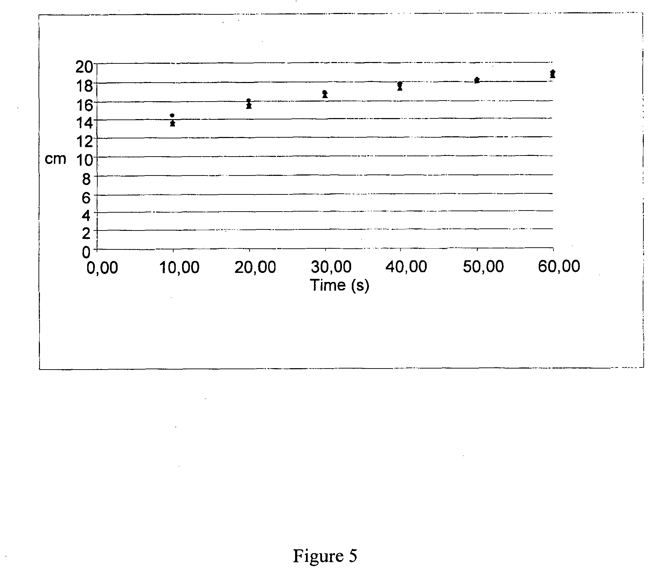

Fluidity measurements were performed with 45 ml sample in a Bostwick consistometer 24925-000 (Christison Particle Technologies Ltd, UK). The fluidity is measured as the distance the sample flows in a track during 60 seconds, with readings taken every 10 s. The results for the WWB-gg bread are shown in figure 5. The results are also shown as arbitrary Bostwick units (distance at t= 60 s/sample volume in ml) in Table 1.

**Table 1 Fluidity presented as arbitrary Bostwick units at 60 s for WWB-gg bread.**

| Arbitrary Bostwick units | [cm/ml] |
|---|---|
| Small intestine | 0,42 |
| Stomach | 0,41 |
| Mouth | 0,41 |

The results show that the flow velocity for the WWB-gg product was similar prior to, and after simulated chewing, stomach or small intestinal conditions.

### Example 4. Viscosity of the bread

Rheology analysis was performed using Bohlin Visco 88 BV, analysis system C30 (system 3).

Three sample solutions were prepared as for analysis of fluidity (see Example 3). The measurements were performed at 37°C.

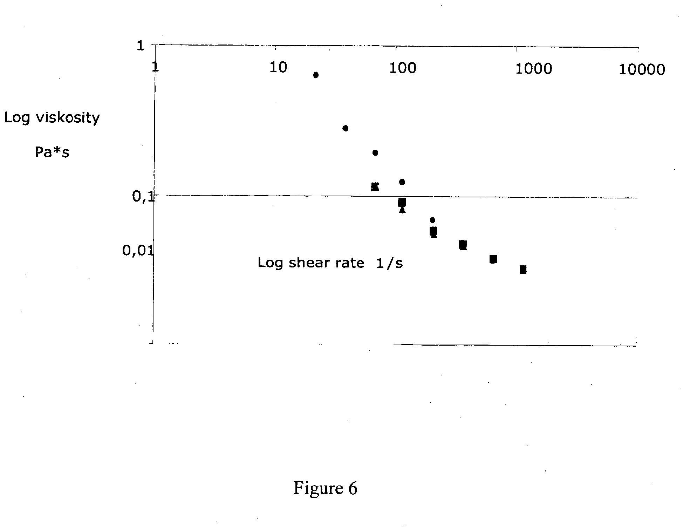

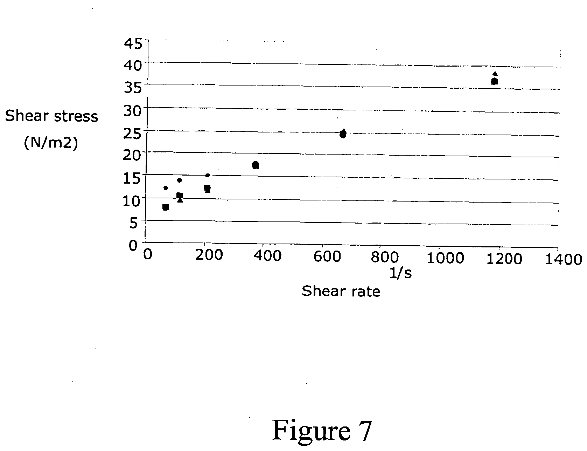

The results for the WWB-gg product are shown as viscosity curves (figure 6) and flow curves (figure 7).

### Example 5. Comparison of fluidity of WWB-gg and guar gum fibre.

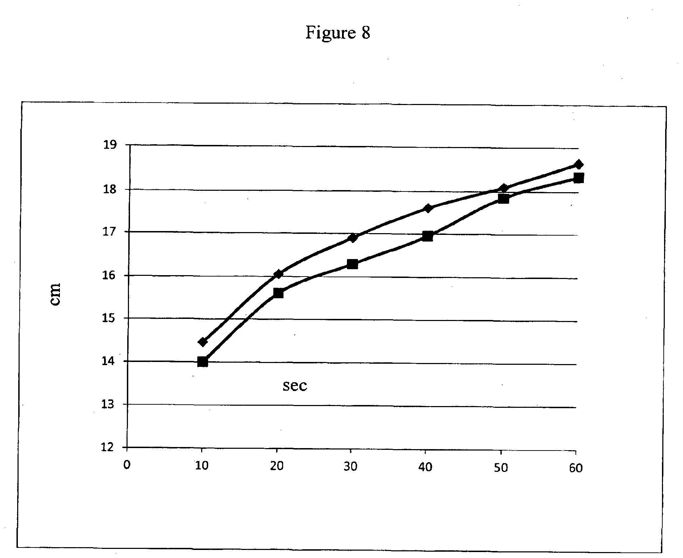

The WWB-gg product suspended in water was compared to a solution of isolated guar gum in water without bread matrix. The results (see Figure 8) show that the fluidity of bread dissolved in liquid (comparable to the situation in the mouth) can be mimicked by guar gum by itself in solution. The concentration of the fibre may be adjusted according to the matrix or food product chosen, for example this may depend on whether it is a bread or bar. See above for examples of food products.

The WWB-gg sample was prepared as in Example 3.

Guar gum was prepared by dissolving 0,45 g guar gum in 100 ml water. Fluidity measurements were performed with 45 ml sample in a Bostwick consistometer 24925-000 (Christison Particle Technologies Ltd, UK). The fluidity is measured as the distance the sample flows in a track during 60 seconds, with readings taken every 10 s. The results are also shown as arbitrary Bostwick units (distance at t= 60 s/sample volume in ml).

### Example 6: Comparisons of fluidity of different dietary fibres.

Samples were prepared by dissolving the fibre in 100 ml water. The fluidity was measured in the same method as described in Example 5 using 45 ml samples. The fibres tested were:
Guar gum: Ceramahl F21, guar gum 5000 cps/200 mesh, C.E. Roeper GmBH Hamburg, Germany; Xanthan Ziboxan F40 from Deosen Corporation Ltd, Shandong, China; Pectin APA 200 LM Amidated Pectin from Yantai Andre pectin Co. Ltd, Shandong, China; Genugel Carrageenan type CJ, from CP Kelco, Skensved, Denmark; Betaglucan (24%) from Lyckeby Stärkelsen, Kristianstad, Sweden; Psyllium 99% 100 mesh Pysillium Husk known as Blond Psyllium or Indian Psyllium from C.E. Roeper GmbH; Alginate K3B426 from IMCD UK Limited. Fluidity was measured as described in Example 3 and Example 5. The results (shown in Table 2) indicate that the fluidity of the dietary fibre samples are influenced by their concentration. The results also demonstrate that a range of fluidities encompassing that of guar gum can be achieved using other dietary fibres, and thus that the fluidity achieved using guar gum can also be achieved using other dietary fibres. The results show that an increasing concentration of xanthan gum leads to a decreasing fluidity, even lower than for guar gum, see Table 2. Pectin may be used to achieve a very similar fluidity to that of guar gum.

Thus the fluidity of the WWB-gg bread in digestive conditions (Table 1) can be achieved by guar gum in water (Table 2), and the fluidity of guar gum can be achieved using other dietary fibres (see Table 2).

**Table 2. Fluidity presented as Arbitrary Bostwick units at 60 s of dietary fibres.**

| Fibre | Fluidity in Arbitrary Bostwick Units | | | | |
|---|---|---|---|---|---|
| Guar gum | | 0,41 (0,45g)* | 0,39 (0,5g) | 0,37 (0,6g) | 0,36 (0,65g) |
| Xanthan | | 0,37 (0,20g) | 0,34 (0,25g) | 0,33 (0,35g) | |
| Pectin | 0,49 (1,5g) | 0,42 (3,0g) | | | |
| Carrageenan | 0,49 (1,5g) | 0,43 (2,0g) | 0,32 (2,5g) | | |
| Beta glucan | | 0,43 (4,0g) | 0,39 (4,5g) | 0,37 (5,0g) | |
| Psyllium | 0,47 (0,75g) | 0,42 (0,9g) | 0,39 (0,95g) | | |
| Alginate | | 0,42 (1,1g) | | | |

| | | | | | |
|---|---|---|---|---|---|
| *The figures in parentheses refer to the amount of the fibre that was dissolved in 100 ml water and tested. | | | | | |

## Claims

1. A food product for use in preventing cognitive dysfunction secondary to Insulin Resistance and Insulin Resistance Syndrome in a subject, which upon ingestion leads to an elevation in delta blood glucose level of >0 mmol/L in the interval 120 mins to 240 mins in the post-prandial phase, the food product having a GI below 70 using a white wheat bread reference, and comprising viscous dietary fibre capable of reducing the rate of digestion and adsorption of carbohydrates in the gastrointestinal tract.

2. The food product for use according claim 1, wherein the food product comprises 5-35%, such as 10-20% viscous dietary fibre by dry weight.

3. The food product for use according to claim 1 or 2, wherein the viscous dietary fibre is selected from the group consisting of pectins, beta-glucans, gums such as xanthan gum, guar gum and carob gum, arabinoxylans, alginates, glucomannans including konjacmannan, psyllium and carrageenans, and/or combinations thereof.

4. The food product for use according to claim 3, wherein the viscous dietary fibre is guar gum.

5. The food product for use according to claim 1, wherein the food product comprises 15% guar gum by dry weight.

6. The food product for use according to any of the preceeding claims wherein the food product is selected from the group consisting of soft bread, crisp bread and flatbread, such as white bread, rye bread, whole wheat bread, whole grain bread or combinations thereof, cakes, buns, biscuits, pizza, pies, sauces, dressings, breakfast cereal products such as flakes and muesli, porridges, pasta, yoghurts, gel foods, snacks, bars, smoothies, shots, probiotic products, soups, drinks, drink or soup mix powders, shakes, fruit purees, composite ready-to- eat meals, lasagne, crepes, pancakes, flatbread wraps, pirogues, purees, jams, marmalades, puddings, jellies, mashed potatoes, and gratin or casseroles, for example potato gratin, broccoli gratin.

7. The food product for use according to any of the preceding claims, wherein the food product is a bread.

8. The food product for use according to claim 1, wherein the food product is a cereal-based bread comprising guar gum.

## Patentansprüche

1. Nahrungsmittel zur Verwendung bei der Verhinderung einer kognitiven Dysfunktion als Folge einer Insulinresistenz und eines Insulinresistenzsyndroms in einem Patienten, das nach der Aufnahme zu einer Erhöhung des Delta-Blutzuckerspiegels von > 0 mmol/L in der Zeitspanne von 120 Minuten bis 240 Minuten in der postprandialen Phase führt, wobei das Nahrungsmittel einen GI unter 70 unter Verwendung einer Weißbrotreferenz aufweist und viskose Nahrungsfaser umfasst, welche die Verdauungsgeschwindigkeit und Adsorption von Kohlenhydraten im Magen-Darm-Trakt reduzieren kann.

2. Nahrungsmittel zur Verwendung nach Anspruch 1, wobei das Nahrungsmittel 5-35 % umfasst, wie beispielsweise 10-20 % viskose Nahrungsfaser bezogen auf das Trockengewicht.

3. Nahrungsmittel zur Verwendung nach Anspruch 1 oder 2, wobei die viskose Nahrungsfaser aus der Gruppe bestehend aus Pektinen, Beta-Glucanen, Gummis wie beispielsweise Xanthangummi, Guargummi und Johannisbrotkernmehl, Arabinoxylanen, Alginaten, Glucomannanen einschließlich Konjakmannan, Psyllium und Carrageenen und/oder Kombinationen derselben ausgewählt wird.

4. Nahrungsmittel zur Verwendung nach Anspruch 3, wobei die viskose Nahrungsfaser Guargummi ist.

5. Nahrungsmittel zur Verwendung nach Anspruch 1, wobei das Nahrungsmittel 15 % Guargummi bezogen auf das Trockengewicht umfasst.

6. Nahrungsmittel zur Verwendung nach einem der vorstehenden Ansprüche, wobei das Nahrungsmittel aus der Gruppe bestehend aus weichem Brot, Knäckebrot und Fladenbrot, wie beispielsweise Weißbrot, Roggenbrot, Vollkornweizenbrot, Vollkornbrot oder Kombinationen derselben, Kuchen, Brötchen, Keksen, Pizza, Pasteten, Soßen, Salatsoßen, Frühstückszerealienprodukten wie beispielsweise Flocken und Müsli, Haferbrei, Nudeln, Joghurts, Gelnahrungsmitteln, Snacks, Riegeln, Smoothies, Sprossen, probiotischen Produkten, Suppen, Getränken, Pulver zum Mischen von Getränken oder Suppen, Shakes, Fruchtpürees, zusammengesetzten Fertigmahlzeiten, Lasagne, Crepes, Pfannkuchen, Fladenbrotwraps, Piroggen, Pürees, Konfitüren, Marmeladen, Puddings, Gelees, Kartoffelpüree und Gratins oder Aufläufen, zum Beispiel Kartoffelgratin, Brokkoligratin ausgewählt wird.

7. Nahrungsmittel zur Verwendung nach einem der vorstehenden Ansprüche, wobei das Nahrungsmittel Brot ist.

8. Nahrungsmittel zur Verwendung nach Anspruch 1, wobei das Nahrungsmittel ein getreidebasiertes Brot ist, das Guargummi umfasst.

## Revendications

1. Produit alimentaire pour une utilisation dans la prévention du dysfonctionnement cognitif consécutif à l'insulinorésistance et au syndrome d'insulinorésistance chez un sujet, qui, lors de l'ingestion, conduit à une élévation du niveau de delta glycémique de > 0 mmol/L dans l'intervalle allant de 120 min à 240 min dans la phase post-prandiale, le produit alimentaire ayant un GI inférieur à 70 à l'aide d'une référence de pain blanc, et comprenant une fibre alimentaire visqueuse capable de réduire la vitesse de digestion et d'adsorption de glucides dans le tractus gastro-intestinal.

2. Produit alimentaire pour une utilisation selon la revendication 1, dans lequel le produit alimentaire comprend 5-35 %, tel que 10-20 %, de fibre alimentaire visqueuse en poids sec.

3. Produit alimentaire pour une utilisation selon l'une des revendications 1 ou 2, dans lequel la fibre alimentaire visqueuse est choisie dans le groupe consistant en pectines, bêta-glucanes, gommes telles que la gomme de xanthane, la gomme de guar et la gomme de caroube, arabinoxylanes, alginates, glucomannanes comprenant le konjacmannane, le psyllium et les carraghénanes, et/ou leurs combinaisons.

4. Produit alimentaire pour une utilisation selon la revendication 3, dans lequel la fibre alimentaire visqueuse est la gomme de guar.

5. Produit alimentaire pour une utilisation selon la revendication 1, dans lequel le produit alimentaire comprend 15 % de gomme de guar en poids sec.

6. Produit alimentaire pour une utilisation selon l'une quelconque des revendications précédentes, dans lequel le produit alimentaire est choisi dans le groupe consistant en pain de mie, pain croustillant et pain plat, comme le pain blanc, le pain de seigle, le pain de blé complet, le pain aux céréales complètes ou leurs combinaisons, gâteaux, petits pains, biscuits, pizzas, tartes, sauces, assaisonnements, produits à base de céréales pour le petit-déjeuner tels que les flocons et le muesli, porridges, pâtes, yaourts, aliments gélifiés, collations, barres, boissons fouettées, petits verres, produits probiotiques, soupes, boissons, mélanges de poudres pour boissons ou soupes, boissons frappées, purées de fruits, repas composites prêts à consommer, lasagnes, crêpes, pancakes, sandwichs roulés à pain plat, pierogi, purées, confitures, marmelades, puddings, gelées, purées de pommes de terre, et gratins ou ragoûts, par exemple gratin de pommes de terre, gratin de brocolis.

7. Produit alimentaire pour une utilisation selon l'une quelconque des revendications précédentes, dans lequel le produit alimentaire est un pain.

8. Produit alimentaire pour une utilisation selon la revendication 1, dans lequel le produit alimentaire est un pain à base de céréales comprenant de la gomme de guar.
